(19) Europäisches Patentamt / European Patent Office / Office européen des brevets

(11) **EP 2 980 625 A1**

(12) **EUROPEAN PATENT APPLICATION**

(43) Date of publication:
03.02.2016 Bulletin 2016/05

(21) Application number: 15179172.0

(22) Date of filing: 30.07.2015

(51) Int Cl.:
*G02B 21/00* (2006.01)　　*A61B 1/00* (2006.01)
*A61B 5/00* (2006.01)　　*G01J 3/18* (2006.01)
*G01J 3/02* (2006.01)　　*G01J 3/28* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: 31.07.2014 GB 201413600

(71) Applicant: **TECHNION RESEARCH AND DEVELOPMENT FOUNDATION, LTD.**
**Haifa 32000 (IL)**

(72) Inventors:
• YELIN, Dvir
　Haifa 45789 (IL)
• BAR-ILAN, Yair
　Ma'alot 21501 (IL)

(74) Representative: **MERH-IP Matias Erny Reichl Hoffmann**
**Patentanwälte PartG mbB**
**Paul-Heyse-Strasse 29**
**80336 München (DE)**

(54) **SPECTRAL IMAGING USING SINGLE-AXIS SPECTRALLY DISPERSED ILLUMINATION**

(57) A technique for spectral imaging using a two-dimensional illumination pattern having spectral dispersion in one axis. The spectral imaging method involves the use of spectrally dispersed illumination, thereby allowing the use of higher intensity source illumination than prior art spectral encoding methods, thus providing high-speed, high-resolution acquisition of spectral data from specimens that cannot tolerate high illumination intensities or that require fast imaging for avoiding motion artifacts. The technique is demonstrated by capturing spectral data cubes of a finger using short exposure durations and a high signal-to-noise ratio.

*FIG. 5*

EP 2 980 625 A1

**Description**

FIELD OF THE INVENTION

The present invention relates to the field of spectral imaging of samples, especially using wide field of view scanning and imaging techniques that cover a large sample area, thereby enabling lower noise imaging at higher speeds.

BACKGROUND OF THE INVENTION

The optical spectrum emitted from a specimen carries invaluable information on its structure, its chemical composition and physical parameters. Spectral imaging, a combination of imaging and spectroscopy, provides three dimensional data sets which contain the spectra from all the points on the imaged object. Spectral imaging has been shown useful for a wide variety of applications, including earth sciences, oceanography, homeland security, and the food industry, as well in biological and clinical applications.

The main challenge of spectral imaging, however, is the acquisition, in a timely manner, of the large three-dimensional data sets that may comprise high-resolution spectra within high-pixel-count images. A variety of techniques had been proposed for effective spectral imaging, including wide field imaging under different wavelength illumination, point and line scanning, and full-frame interferometric Fourier spectroscopy. Optical microscopes generally offer at least one of these modalities for spectral imaging. In US Patent Application Publication US2012/0025099 for "Systems and Methods for Spectrally Encoded Imaging" (now US Patent No. 9,046,419), having a co-author common with the present application, there is described the possibility of performing spectrally encoded endoscopy for capturing spatially resolved spectra by using a two-dimensional scanning of a spectral line across a sample, the back-scattered light being transmitted through an optical fiber and analyzed by a fast spectrometer. This method had a superior signal-to-noise ratio (SNR) compared to point and line scanning and could potentially be useful for various clinical endoscopic applications.

The recent advance in light source technology had provided a range of high-brightness ultra-broadband light sources; examples of these technologies include supercontinuum light generation in fibers and in vacuum. For many imaging applications, and especially for biological samples, there are however, strict limits to the irradiance levels that a given sample could tolerate. In most biomedical applications, for example, a maximum permissible exposure (MPE) levels exist for every tissue type, above which the excitation light would alter the properties of the specimen or induce a long-term damage. When using focused illumination for the imaging, the MPE levels are quickly reached without the ability to use currently available high intensity light sources, and their concomitant advantages in increasing the SNR of spectral imaging.

There therefore exists a need for a spectral imaging system and method which overcomes at least some of the disadvantages of prior art systems and methods.

The disclosures of each of the publications mentioned in this section and in other sections of the specification, are hereby incorporated by reference, each in its entirety.

SUMMARY

The present disclosure describes new exemplary systems and methods for efficient, high signal-to-noise ratio, two-dimensional spectral imaging, using wide-field, spectrally dispersed illumination. The technique uses a two-dimensional illumination pattern having spectral dispersion in one axis, which is mechanically scanned relative to the sample, along that dispersion direction. The image data is collected using a two-dimensional video camera, such that parallel processing of the spectral image data at the frame rate of the camera is enabled. This allows much simpler signal processing to be performed than for the high serial data rates obtained from prior art spectral encoding imaging techniques using two dimensional scanning techniques. Because of the comparatively large area over which the illumination is spread, the method allows high-speed, high-resolution acquisition of spectral data from specimens that cannot tolerate high illumination intensities, or from specimens that require fast imaging for avoiding motion artifacts.

The spectral dispersion along the scanning axis may most conveniently be generated by use of a diffraction grating, and the lateral spread of the beam in the orthogonal direction, to generate the large illumination area, may be advantageously obtained using a cylindrical lens oriented to focus the incident beam down in the dispersion direction, but not in the direction orthogonal thereto. Although the method is most conveniently performed using orthogonal dispersion and non-focused directions, it is to be understood that this is not a strict requirement, but that other angles may also be used if deemed more useful, and that this disclosure is not intended to limit the methods and systems to orthogonally disposition. The camera may be a monochromatic camera and the single-axis scanning of the sample may be performed either by motion of the system, or of the sample, or of both.

Thus, by illuminating a large area and detecting spectrally encoded reflectance from an entire sample plane, spectral imaging is efficiently conducted at low irradiance levels and without the need for rapid two-dimensional scanning and high data rate signal processing.

There is thus provided in accordance with an exemplary implementation of the methods described in this disclosure, a method for performing spectral imaging of a target sample, comprising:

(i) providing a beam of illumination having a range of spectral intensities,
(ii) spectrally dispersing the beam in a first direction, such that the dispersed beam is spectrally spread along the target sample,
(iii) focusing the spectrally dispersed beam onto the target sample only in the first direction, such that the dispersed beam illuminates a two dimensional area of the target sample, and
(iv) imaging the target sample in two dimensions as the illumination beam is scanned relative to the sample in the first direction.

Such a method may comprise the further step of assembling a spectral cube incorporating also the spectral data for each imaged location. Additionally, the illumination of a two dimensional area of the target sample enables the use of a higher intensity illumination source than with spectrally encoded serial imaging, without engendering damage to the target sample. In any of the above described methods, the parallel imaging of an entire area of the target sample both enables faster scans to be achieved than with spectrally encoded serial imaging, and enables a higher signal to noise ratio image to be obtained than with spectrally encoded serial imaging. In some implementations of such a method, the step of spectrally dispersing the beam is performed using a diffraction grating, while in others, the step of focusing the spectrally dispersed beam onto the target sample only in the first direction is performed using a cylindrical lens. Furthermore, the imaging may be performed monochromatically.

Other implementations of the present disclosure may further involve a system for performing spectral imaging of a target sample, comprising:

(i) a broadband illumination source, optically manipulated such that it produces a generally collimated beam,
(ii) a spectral dispersing element, aligned such that the beam is spectrally spread along the target sample in a first direction,
(iii) a cylindrical lens disposed and oriented such that the spectrally dispersed beam is focused onto the target sample only in the first direction, such that the dispersed beam illuminates a two dimensional area of the target sample, and
(iv) a two dimensional imaging array disposed such that it captures two dimensional images of the target sample as the illumination beam is scanned relative to the sample in the first direction.

In such a system, the illumination source may have a higher intensity than a source for use in spectrally encoded serial imaging, without engendering damage to the target sample, because of the use of illumination of a two dimensional area of the target sample. Additionally, the spectral dispersing element is a diffraction grating. Finally, the two dimensional imaging array may be a monochromatic array.

BRIEF DESCRIPTION OF THE DRAWINGS

The present invention will be understood and appreciated more fully from the following detailed description, taken in conjunction with the drawings in which:

Fig.1 illustrates schematically a representation of a prior art spectrally encoded imaging system;
Fig. 2 illustrates schematically the scanning scheme used in the prior art methods of Fig. 1;
Fig. 3 illustrates schematically the novel scanning scheme for performing spectral imaging, as described in the present disclosure, using only a single scan direction;
Fig. 4 illustrates schematically using a simple sample target, how the spectral imaging scheme of Fig. 3 operates;
Fig. 5 illustrates schematically, an exemplary system enabling the spectral scanning of the present disclosure to be implemented; and
Fig. 6 shows an example of spectral imaging of live tissue, in this case a finger, in order to illustrate the resolution obtainable using the exemplary system of Fig. 5.

DETAILED DESCRIPTION

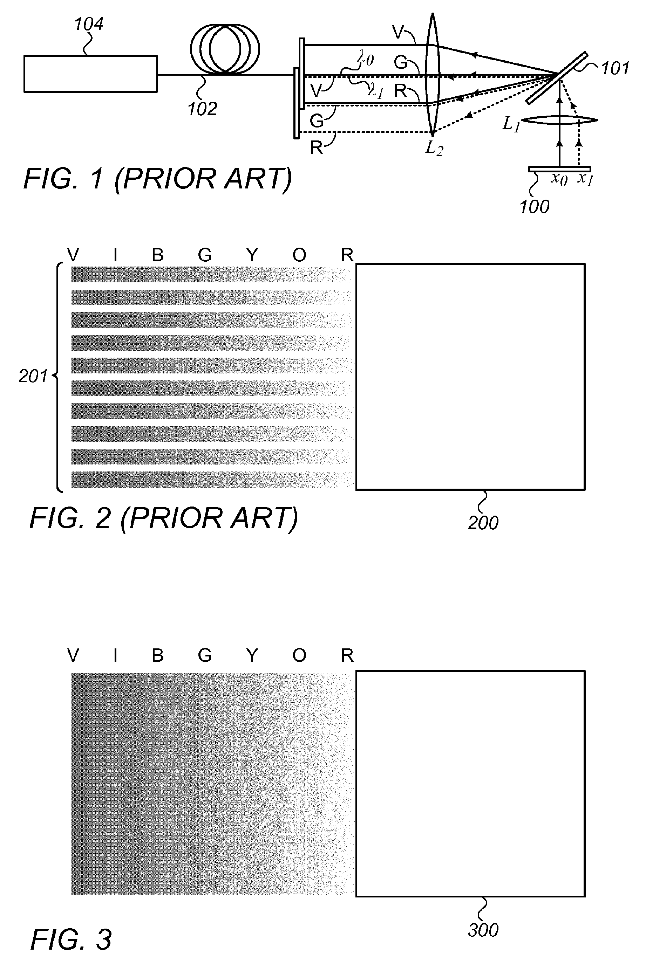

Reference is first made to Fig. 1, which illustrates schematically a representation of a prior art spectrally encoded imaging system, such as is shown in Figs. 1A and 1B of the above referenced US patent No. 9,046,419. The apparatus shown therein is described for use in imaging a sample surface by using a two-dimensional combination of scanning motion, such as for incorporation into an endoscopic probe. In Fig. 1, showing the detection section of the system, the continuous line depicts light scattered from a certain segment $x_0$ of a sample 100 and diffracted by the diffractive element 101, while the dotted lines depict light scattered from an overlapping segment $x_1$ of the sample and also diffracted by the diffractive element 101. The optical assembly comprising the diffraction grating 101 and its associated lenses $L_1$ and $L_2$ is maneuvered, either by tilting the grating or by manual motion, to capture the multi wavelength data, depicted in Fig. 1 as $\lambda_0$ and $\lambda_1$, according to scanning patterns which are set so that a desired number of the wavelengths are captured from each pixel in the image plane of the sample. The collected spectrally encoded light may be transferred by a fiber 102 to a spectral analysis unit 104.

Typically, the illuminating beam is focused by means of a spherical lens down to its scanning spot size, and is scanned across the entire surface of the sample by means of a sequential scanning raster, with typically a rapid horizontal scan in conjunction with a slow vertical scan, such that each point of the sample is sequentially scanned. Such a scanning procedure is shown in Fig. 2, where the sample is depicted as the square area 200, and the scans 201 of the complete raster are shown on the left of the drawing. Each horizontal scan, from left to right in the drawing, is performed using the spectrally dispersed source illumination. The source illumination output from the optical fiber is collimated, spectrally dispersed typically by means of a diffraction grating, and focused using a spherical lens such that it is spectrally spread out as a scanning spot moving in the horizontal direction, which, for this example, has the red wavelengths at the right hand side of the dispersed beam, and the violet at the left hand side. For convenience, the colors are depicted by the initial letters of the conventionally designated names of the visible spectrum - Red, Orange, Yellow, Green, Blue, Indigo and Violet. The width (in the vertical dimension in Fig. 2) of each scanned line is a function of the spot size of the focused beam. The spread of the beam in the horizontal direction is a result of the spectral dispersion. Therefore each point of the sample covered by the horizontal scanning motion is scanned sequentially by the dispersed wavelengths of the illuminating light, such that each point on the first horizontal line to be scanned of the sample is illuminated sequentially at each wavelength of the dispersed incident light, beginning at the red and ending at the violet (if all of the visible range is included). The signals detected from each point in that horizontal line represents light scattered sequentially from illumination at each wavelength of the scattered light, and can be spectrally analyzed by one of the methods mentioned in the Background section hereinabove, to generate the serial output data stream representing the image. Once the first horizontal spectral scan is complete, the scanner moves vertically down by one row, and the horizontal scanning process is repeated for the second row, and so on until the whole of the sample area has been sequentially spectrally scanned. The scanning can also be performed using the alternative scanning directions, such that the fast scan is performed vertically, imaging a complete column with each pixel down the column being imaged, followed by slow horizontal motion, such that each pixel is now illuminated by another sequential wavelength of the dispersed illumination, and so on until the entire sample area has been scanned by all of the wavelengths. Using either of these schemes, the result is a complete two-dimensional scan of the sample area, carried out sequentially by all of the dispersed wavelengths of the illuminating light, such that when including the spectrally scanned data, a three-dimensional image data cube is obtained, two dimensions representing the two-dimensional spatial scan and the third dimension representing the wavelength of illumination at each scanned location.

Since the illuminating wavelength of any lateral and longitudinal point of the scanned target is known at every moment in time, the detected wavelength can be used to register the position being illuminated at each pixel and pixel, since at every point of time, the illuminating wavelength at each pixel is known. This spectrally spread scanning procedure is thus essentially a spectrally encoded imaging scheme, and the signal received in the detector fiber can be related by means of its wavelength to the position on the target from which that signal was received.

Reference is now made to Fig. 3, which illustrates, in contrast to the above-described spectrally encoding scheme, a novel scanning scheme for performing spectral imaging, as described in the present disclosure. This scheme requires only a single scan direction, with the image information in the orthogonal direction being acquired by virtue of the detection being performed on a two-dimensional imaging device such as a CCD or CMOS video camera, in contrast to the prior art where a single channel detection scheme was used. In the present disclosure, taking the dispersion direction for the example shown in Fig. 3 to be arbitrarily the horizontal direction, as it was in the prior art configuration of Fig. 2, the horizontally dispersed illuminating beam is spatially spread out over the height of the sample area, such that as the dispersed beam is scanned in the horizontal direction, a complete vertical row of locations on the sample is illuminated with sequentially changing wavelength light, but each vertical row has the same illumination wavelength at any point in time. The camera images the entire sample area 300 at its characteristic frame rate, typically at a rate of up to some tens of frames per second for commonly available cameras of economically acceptable cost, and the information on each successive frame is composed of a two dimensional lateral matrix of one dimensional columns, each column having sequentially changing illumination wavelengths arising from the laterally scanned dispersed illumination, thereby providing the spectral imaging characteristics of the method. This scheme enables substantially faster imaging data to be acquired, with the simplicity of only needing a single scanning direction. Thus, even though a monochromatic camera can be used, each pixel defines the position and wavelength of the corresponding position in the sample area, since the lateral incremental mechanical scan of the dispersed light is correlated to the spatial dispersion of the illumination. In addition, the large two dimensional illumination area allows the use of high power light sources, since when such a high power source is spread over a large area, the irradiance (light power divided by the surface area) is small, lowering potential damage to the sample. This method is thus optimal for applications that are limited by a maximum permitted irradiance levels.

The scanning scheme is implemented by using separate elements for spreading out the illuminating beam in the two selected directions, which may be most conveniently chosen to be orthogonal. The illumination may be spectrally dispersed by a diffraction grating in the horizontal direction, which is the mechanical scanning direction, and spatially spread in the other, vertical, direction, such as by use of a cylindrical lens. Consequently, the wavelength of the illumination varies sequentially over the horizontal direction, which is the mechanical scanning direction, but is constant down each column of pixels in the vertical direction. The signal processing circuitry of the camera can then synchronize the data from sequential frames, each imaging a successive horizontal position of the mechanical scan, and can thereby generate a spectral image of the scanned target area substantially more quickly than the prior art spectral encoding methods.

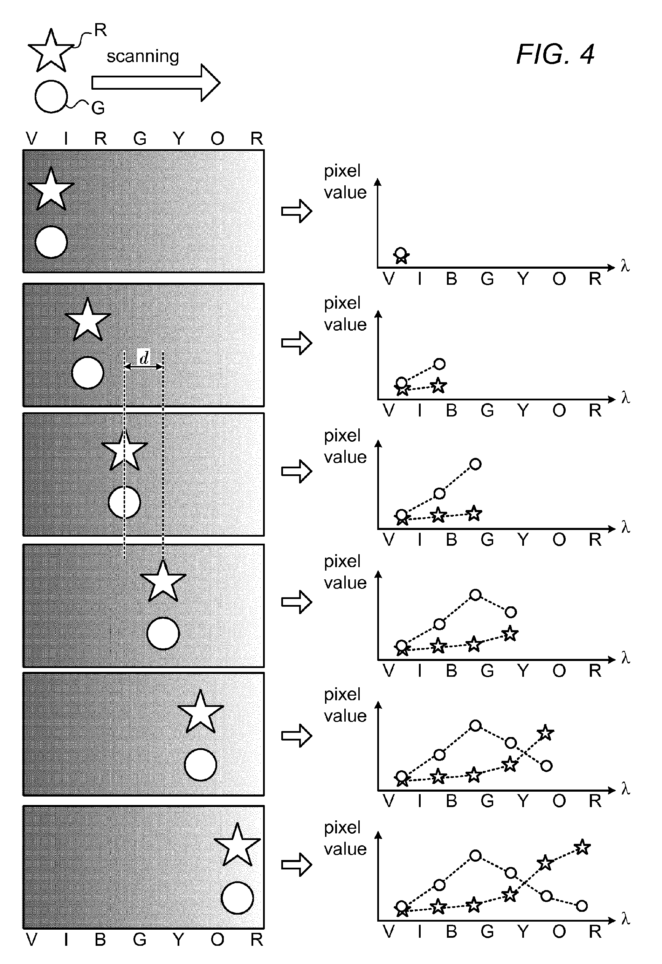

Reference is now made to the drawings of Fig. 4, which illustrate schematically using a simple sample target, how this spectral imaging scheme operates. Spectral data acquisition is conducted by repeatedly capturing images at the frame rate of the camera, typically, 15 to 20Hz, though faster frame rates can be obtained with more costly cameras, while the sample is translated along the spectral x-axis, such as by using a motorized linear translation stage. Once a full scan is completed, a spatial-spectral data cube is assembled by digitally stacking the camera images with a small shift (d) that corresponds to the distance that the sample had moved between subsequent frames. The formation of the spatial-spectral cube is schematically illustrated in Fig. 4, depicting the gradual traverse of two exemplary features across the illumination spectral pattern. As each sample location passes through different illumination wavelengths, λ, shown on the abscissa of the graphs of Fig. 4, its reflectance varies according to its own characteristic reflection spectrum, resulting in a gradual capture of the full spectrum from each resolvable sample location.

Fig. 4 is based on a sample made up of a star and a circle, wherein, for the purpose of explaining how this imaging scheme operates, the star is assumed to have a red color, R, reflecting wavelengths of the order of 700 nm, while the circle is assumed to be green, G, reflecting typically in the region of 550 nm. For the purposes of simplifying this explanation, the colors R, O, Y, G, B, I, and V are used to designate the different wavelengths of the dispersed illumination, both in the depiction of the scan, and on the graphs of Fig. 4. The dispersed illumination is shown to range from the violet V at the left hand side of the dispersed illumination to the red R or near infra-red at the right hand side. Each successive imaging event shows the samples moved by an interval d across spectrally dispersed illumination. The pixel output from the imaging camera is plotted on the graphs on the right hand side of Fig. 4, as a function of the spectrally resolved wavelength λ. As is observed, as the scan proceeds, the camera pixel output for the intensity of each of the two target samples, follows the reflected light expected from the relationship between the color of the sample, and the color of the illumination through which the samples are passing at the time the output is measured. Thus, the reflection goes up in accordance with the wavelength of the illuminating beam relative to the spectral reflectance of the sample object. Thus, in the uppermost drawing, the red star R and the green circle G are situated in the violet range of the illumination, such that almost no light is reflected from either, as shown in the top graph. In the second drawing, the sample containing the star and circle has moved to the right and the two objects are now entering the blue-green range of the illumination, such that the red star still has little reflected output, while the green circle does begin to show a significant reflected signal, as seen in the graph to the right of the second drawing. In the third graph, the green circle is being illuminated by the green part of the spectrum, and so shows maximum reflected light, while the red star still reflects almost none of the green light. Finally, when the scan is nearing completion, as shown in the bottom graphs, it is seen that the green circle output signal has peaked in the central green region of the detected spectrum, and reflects almost nothing in the violet end of the spectrum, while the red star image has peaked at the long wavelength, red end of the output spectrum.

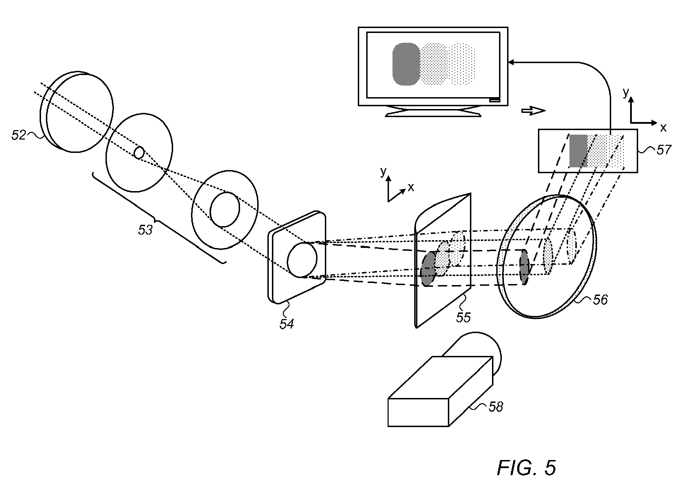

Reference is now made to Fig. 5, which illustrates schematically, an exemplary system, enabling the spectral scanning methods of the present disclosure to be implemented. The system contains an illumination channel and an imaging channel. The illumination source may conveniently be a spatially coherent light beam from a broadband supercontinuum source, such as the model SC-400, supplied by Fianium Ltd. of Southampton, UK. A lowpass filter 52 may be used in order to define the spectral region to be used - for example, infra-red wavelengths above 800 nm may be blocked in order to increase the signal-to-noise ratio if the visible region is being used for the spectral imaging, as is commonly done using commonly available imaging cameras. The illumination may then be passed through a beam expanding telescope 53, optionally made up of two achromatic lenses, to improve the resolution obtainable from the system. The expanded collimated beam is then passed through the diffraction grating 54, which, in the example system shown in Fig. 5, disperses the light in a horizontal (x) direction. In the example of Fig. 5, only three different colors are shown, as depicted by the different shading of three beams diffracted by the diffraction grating, but it is to be understood that in practice, a continuum of wavelengths is generated by the dispersion process. A grating having 600 lines/mm may typically be used for the resolution required to accurately image samples typical of body-part sizes. The dispersed light is then focused by a cylindrical lens 55, aligned such that the light is focused along the same horizontal (x) axis as the dispersion direction. In the orthogonal y direction, since the lens is cylindrical, there is no focusing, such that the beam keeps its original height. The result at the focal plane of the cylindrical lens is an area of spectrally dispersed illumination having constant wavelength over the height of any vertical line, but varying wavelengths along the dispersion direction x. The spectrally dispersed illumination may then be passed through a beam splitter 56, which directs the incident illumination onto the sample 57 to be spectrally imaged. The resulting spectrally dispersed illumination pattern shown in Fig. 5 is a rectangle spanned by the diffraction grating in the horizontal (x) axis, and the non-focusing dimension of the cylindrical lens along the vertical (y) axis. The light reflected from the sample may be imaged through the beam splitter 56 using a high-resolution monochrome camera 58, typically having of the order of 5M pixels. Lateral resolution of approximately $17\mu$m can be thus obtained over a field of view covering 16.5 mm by 10.3 mm of the sample area. The mechanical scanning in the x direction can be achieved either by motion of the sample or by motion of the whole system relative to the sample.

Since the illumination is spread over the entire sample area, it is possible to use sources of much higher intensity than that used in the prior art spectral encoding scanning schemes, without the local illumination intensity being of a magnitude that may cause tissue damage or burns. This feature is important in that it enables a higher signal-to-noise ratio to be obtained than that of the prior art schemes.

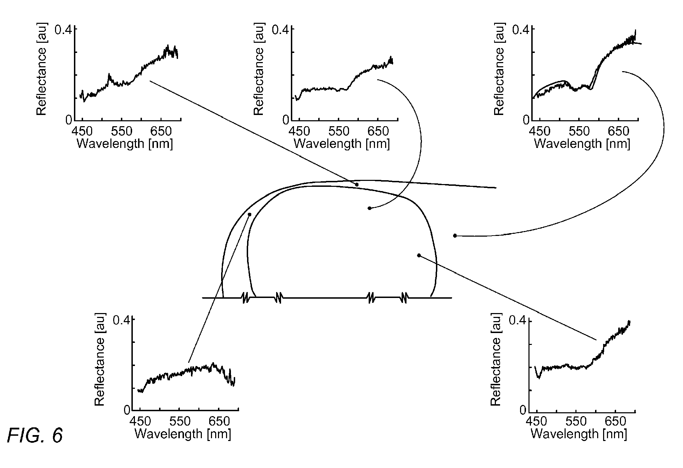

In order to illustrate the advantage of the systems described in this disclosure over previously available spectral imaging schemes, reference is now made to Fig. 6 which shows an example of the resolution obtainable using the exemplary system of Fig. 5. Fig. 6 shows an example of spectral imaging of live tissue, in this case a finger. The finger in Fig. 6 is a schematic representation of a true-life scanned finger. The finger was scanned in the x-axis at a velocity of 0.6 mm/s and imaged at a rate of 15 frames/sec. The raw data set was comprised of 1100 overlapping images that result in 600 individual wavelengths measured for each sample location (approximately 0.5 nm wavelength sampling intervals). Total illumination power was 250 mW, resulting in an average irradiance on the finger of approximately 78 mW/cm$^2$. Exposure duration of each frame was only 0.1 msec. and total imaging duration was 73 sec., both of which were limited by the camera electronics. A number of individual spectra of selected locations from the effective 750x1800x600 pixel data cube (x-y-$\lambda$ axes, respectively) are shown in Fig. 6, the location of each plotted spectrum being indicated on a single selected frame of the finger. Some of the spectra within the data cube, especially the spectrum on the top right hand side of Fig. 6, which is imaged from the skin of the finger, clearly show the distinctive spectral pattern of oxy-hemoglobin that is characterized by two reflection dips at 545 nm and 570 nm. For comparison, the oxy-hemoglobin spectrum itself is plotted in the same graph as that of the spectral plot of the skin of the finger, at the top right hand side of Fig. 6, and as is observed, the spectral image of the skin tissue of the finger is very close to the characteristic spectrum of oxy-hemoglobin.

In order to explain why the SNR and resolution of the spectrally dispersed illumination spectral imaging (SDISI) methods described in this disclosure are higher than the prior art point and line scanning techniques, the SNR of the present technique is first derived using the methods and notation derived from the article entitled "Spectrally Encoded Spectral Imaging" by A. Abramov et al, published in Optics Express, Vol. 19, pp. 6913-6922 (2011).

The maximum signal (in electrons) measured for each resolvable element (x,y,$\lambda$) is given by:

$$Q_e r[I_{max}s/(h\nu)]t \qquad (1)$$

where $Q_e$ denotes the detector quantum efficiency,
r denotes sample reflectivity,
$I_{max}$ denotes the MPE in units of W/cm$^2$,
s denotes the area of a single spatial resolvable element,
h is Planck's constant,
v denotes the optical frequency, and
t denotes the exposure time for a single resolvable element.

Since the illumination is spectrally dispersed, each pixel in a single $N \times N$ pixel (square) frame is transiently illuminated by a single wavelength, while the reflected light from that pixel is detected during an exposure time given by:

$$t = T/(N + M) \qquad (2)$$

where T denotes the total data acquisition time and M denotes the number of spectral resolvable elements along the x-axis (N, M >> 1). Assuming that the dark current D is the dominant noise source (neglecting shot and read noise), the SNR can be shown to be given by:

$$\mathrm{SNR}_{\mathrm{DISI}} \cong \frac{\dfrac{Q_e r I_{\max} s}{h\nu} t}{\sqrt{Dt}} = \frac{Q_e r I_{\max} s \sqrt{T}}{h\nu \sqrt{D}} \sqrt{\frac{1}{N+M}}. \tag{3}$$

Assuming, for brevity, M = N, the SNR in Eq. (3) is $N^{1/2}$-times higher than the SNR of the previously reported spectrally encoded spectral imaging (SESI) technique, as described in the above referenced Abramov et al article, and N/√2-times higher than spectral imaging using line scanning, as described in the article entitled "Design, Construction, Characterization, and Application of a Hyperspectral Microarray Scanner" by M.B. Sinclair et al, published in Applied Optics, Vol. 43, pp. 20-79-2088 (2004). In applications that involve high pixel counts, this represents a significant, several-fold improvement in SNR. In the SDISI systems of the present disclosure, however, speckle contrast is relatively high, being approximately 0.1 for a single point-spectrum on the sample. Spatial averaging over several neighboring pixels may be able to reduce speckle noise. The average SNR for imaging the human finger shown in Fig. 6 was found to be approximately 33.5 dB for the spectral data and 27 dB for the image data. These SNRs were achieved using short 0.1 msec. exposure durations for each frame, which is equivalent to acquisition rates of up to nine spatial-spectral data cubes per second. Effective video-rate spectral imaging would thus be achievable using higher frame-rate cameras having comparable levels of dark currents, such as cooled detector arrays. However, such solutions would be substantially more expensive than the use of uncooled CCD or CMOS video camera imagers, as are in common use.

In contrast to most spectral imaging methods, in the currently described SDISI methods, the spatial and spectral resolutions are directly linked - the maximum number of resolvable wavelengths is essentially limited by the number of resolvable points in a single frame. In specific cases where high spectral resolution is not necessary, the physical step-size between frames may be increased, resulting in higher acquisition rates of under-sampled spectra. The challenges toward practical implementation of SDISI are related mainly to the generation of the somewhat complex illumination pattern and to the calibration and alignment procedures of the illumination and the imaging optics. In its current form, SDISI is effective in measuring reflectance, absorption and backscattering from a specimen, but is generally unsuitable for spectral imaging of fluorescence markers, due to the inherent difference between their excitation and emission spectra. Also, compared to prior art spectrally encoded spectral imaging methods, the SDISI of the present disclosure may be less suited for endoscopic applications within narrow ducts, due to the lack of an encoding technique enabling the use of a single fiber feed, and the need to rely on a full 2-dimensional image capturing device, such as a camera array.

It is appreciated by persons skilled in the art that the present invention is not limited by what has been particularly shown and described hereinabove. Rather the scope of the present invention includes both combinations and subcombinations of various features described hereinabove as well as variations and modifications thereto which would occur to a person of skill in the art upon reading the above description and which are not in the prior art.

## Claims

1. A method for performing spectral imaging of a target sample, comprising:

    providing a beam of illumination having a range of spectral intensities;
    spectrally dispersing said beam in a first direction, such that said dispersed beam is spectrally spread along said target sample;
    focusing said spectrally dispersed beam onto said target sample only in said first direction, such that said dispersed beam illuminates a two dimensional area of said target sample; and
    imaging said target sample in two dimensions as said illumination beam is scanned relative to said sample in said first direction.

2. The method of claim 1, comprising the further step of assembling a spectral cube incorporating also the spectral data for each imaged location.

3. The method of claim 1, wherein the illumination of a two dimensional area of said target sample enables the use of a higher intensity illumination source than with spectrally encoded serial imaging, without engendering damage to said target sample.

4. The method of claim 1, wherein the parallel imaging of an entire area of said target sample enables faster scans to be achieved than with spectrally encoded serial imaging.

5. The method of claim 1, wherein the parallel imaging of an entire area of said target sample enables a higher signal to noise ratio image to be obtained than with spectrally encoded serial imaging.

6. A method according to any of the previous claims, wherein said step of spectrally dispersing said beam is performed using a diffraction grating.

7. A method according to any of the previous claims, wherein said step of focusing said spectrally dispersed beam onto said target sample only in said first direction is performed using a cylindrical lens.

8. A method according to any of the previous claims, wherein said imaging is performed monochromatically.

9. A system for performing spectral imaging of a target sample, comprising:

a broadband illumination source, optically manipulated such that it produces a generally collimated beam;
a spectral dispersing element, aligned such that said beam is spectrally spread along said target sample in a first direction;
a cylindrical lens disposed and oriented such that said spectrally dispersed beam is focused onto said target sample only in said first direction, such that said dispersed beam illuminates a two dimensional area of said target sample; and
a two dimensional imaging array disposed such that it captures two dimensional images of said target sample as said illumination beam is scanned relative to said sample in said first direction.

10. The system of claim 9, wherein said illumination source may have a higher intensity than a source for use in spectrally encoded serial imaging, without engendering damage to said target sample, because of the use of illumination of a two dimensional area of said target sample.

11. A system according to either of claims 9 and 10, wherein said spectral dispersing element is a diffraction grating.

12. A system according to any of claims 9 to 11, wherein said two dimensional imaging array is a monochromatic array.

FIG. 1 (PRIOR ART)

FIG. 2 (PRIOR ART)

FIG. 3

FIG. 4

FIG. 5

FIG. 6

Europäisches
Patentamt

European
Patent Office

Office européen
des brevets

# EUROPEAN SEARCH REPORT

Application Number

EP 15 17 9172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YAIR BAR-ILAN ET AL: "Spectral imaging using single-axis spectrally dispersed illumination", OPTICS LETTERS, OPTICAL SOCIETY OF AMERICA, US, vol. 39, no. 17, 30 July 2014 (2014-07-30), pages 5177-5179, XP001591559, ISSN: 0146-9592, DOI: 10.1364/OL.39.005177 [retrieved on 2014-08-27] * page 5177 - page 5178; figures 1,2 * | 1-12 | INV. G02B21/00 A61B1/00 A61B5/00 G01J3/18 G01J3/02 G01J3/28 |
| A,D | US 2012/025099 A1 (YELIN DVIR [IL] ET AL) 2 February 2012 (2012-02-02) * paragraph [0048] - paragraph [0060]; figures 1A, 1B * | 1-12 | |
| A | US 2007/188855 A1 (SHISHKOV MILEN [US] ET AL) 16 August 2007 (2007-08-16) * paragraphs [0007], [0008], [0046], [0047]; figures 1,2,9B,11A * | 1-12 | |
| A | KEVIN K. TSIA ET AL: "Performance of serial time-encoded amplified microscope", OPTICS EXPRESS, vol. 18, no. 10, 10 May 2010 (2010-05-10), page 10016, XP055233514, ISSN: 2161-2072, DOI: 10.1364/OE.18.010016 * page 10019 - page 10021 * | 1-12 | TECHNICAL FIELDS SEARCHED (IPC) G02B A61B G01J |

The present search report has been drawn up for all claims

| Place of search | Date of completion of the search | Examiner |
|---|---|---|
| Munich | 8 December 2015 | Besser, Veronika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 15 17 9172

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2015

| Patent document cited in search report | Publication date | Patent family member(s) | Publication date |
|---|---|---|---|
| US 2012025099 A1 | 02-02-2012 | NONE | |
| US 2007188855 A1 | 16-08-2007 | US 2007188855 A1 | 16-08-2007 |
| | | US 2012328241 A1 | 27-12-2012 |
| | | US 2015045622 A1 | 12-02-2015 |
| | | WO 2007084903 A2 | 26-07-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120025099 A

- US 9046419 B

**Non-patent literature cited in the description**

- **A. ABRAMOV et al.** Spectrally Encoded Spectral Imaging. *Optics Express,* 2011, vol. 19, 6913-6922

- **M.B. SINCLAIR et al.** Design, Construction, Characterization, and Application of a Hyperspectral Microarray Scanner. *Applied Optics,* 2004, vol. 43, 20-79, 2088